Europäisches Patentamt

European Patent Office

Office européen des brevets

(19)

(11) **EP 0 452 299 B1**

(12) **EUROPEAN PATENT SPECIFICATION**

(45) Date of publication and mention
of the grant of the patent:
**18.06.1997 Bulletin 1997/25**

(51) Int Cl.6: **C07K 1/12, A61K 35/30**

(21) Application number: **91890075.4**

(22) Date of filing: **11.04.1991**

(54) **Use of a mixture of peptides and amino acids in the prophylaxis or treatment of dementia**

Verwendung einer Mischung von Peptiden and Aminosäuren für die Prophylaxis oder Behandlung von Dementia

Utilisation d'un mélange de peptides et d'acides amino pour la propylaxe ou le traitement de la demence

(84) Designated Contracting States:
**AT BE CH DE DK ES FR GB GR IT LI LU NL SE**

(30) Priority: **12.04.1990 JP 97207/90
02.08.1990 JP 206249/90**

(43) Date of publication of application:
**16.10.1991 Bulletin 1991/42**

(73) Proprietor: **EBEWE Arzneimittel Gesellschaft mbH
A-4866 Unterach (AT)**

(72) Inventors:
• **Tsurusawa, Yutaka
Sumiyoshi-Ku, Osaka-shi (JP)**
• **Nishiyama, Makito
Kawachinagano-shi, Osaka-fu (JP)**
• **Fujimoto, Michitaro
Tondabayashi-shi, Osaka-fu (JP)**
• **Iwamoto, Nobuyuki
Habikino-shi, Osaka-fu (JP)**
• **Seki, Hirokazu
Kitakatsuragi-gun, Nara-Ken (JP)**

(74) Representative: **Pfeifer, Otto, Dipl.-Ing. et al
Patentanwälte Schütz und Partner,
Fleischmanngasse 9
1040 Wien (AT)**

(56) References cited:
**EP-A- 0 172 987        EP-A- 0 326 075
EP-A- 0 327 769        GB-A- 866 509**

• **MEDIZINISCHE WELT vol. 31, no. 17, 25 April
1980, F.R.G. pages 636 - 637; BAYER E:" über die
Therapie mit dem Hirnhydrolysat Cerebrolysin."**
• **E.: "Über die Therapie mit dem Hirnhydrolysat
Cerebrolysin."**
• **DERWENT ABSTRACT / WPIL AN = 87-262218 &&
SU-A-1286206 [ TASHK MEDICINE INSTITUT ]
Published 30 january 1987.**

**Description**

New pharmaceutical applications will be provided on the basis of the findings that the present invention is related to a bioactive substance which exhibits an activity to enhance neuroaxon production on ganglion cells; in more detail on the basis of the findings that the mixture of peptides prepared by controlled enzymatic decomposition of swine brain protein fraction and amino acids exhibits an activity to enhance neuroaxon production on ganglion cells. In addition, said bioactive substance exhibits an activity as a nerve nutritive factor on the central nerve, in particular, choline-working nerve cells; in more detail, on the basis of the findings that the mixture of peptides prepared by controlled enzymatic decomposition of swine brain protein fraction and amino acid exhibits an activity as a nerve nutritive factor on the central nerve closely related to dementia, in particular, choline-working nerve cells through the administration routes easy for clinical applications, i.e. intravenous, oral, intramuscular or subcutaneous routes.

The mixture of peptides and amino acids in the present invention contains approximately 5 % of peptides with a molecular weight of 10,000 or less and approximately 85 % of free amino acid. This mixture is already in practical use in Austria for the treatment of cerebral vascular metabolism insufficiency, slight infantile atelencephalia, cerebral apoplexy, cerebral contusion, etc. It is a mixture of peptides and amino acids such as alanine, arginine, asparaginic acid, glutaminic acid, glycine, histidine, isoleucine, leucine, lysine, methionine, phenylalanine, proline, serine, threonine, tryptophane, cysteine, valine, and thyrosine.

If changes occur in nerve cells which support cerebrum functions and in fibrous connection thereof, symptoms of dementia appear. Dementia is considered to be induced by the factors of reduction in nerve transmission substances like acetylcholine, serotonin and noradrenalin, change in neurofibril and disappearance of nerve cells.

It is known that when nerve cells are diminished in the temporal lobe, parietal lobe and frontal lobe and when a remarkable change occurs in the part called dendrites which sustain stimuli, the synapse, the junction to which fibers from other nerve cells are attached, is reduced. Such factors as an increase in acetylcholine and nerve growth, in particular neuroaxon production, may lead to an inhibition of occurrence of dementia.

At present, the etiology of dementia is not yet elucidated. Trials are in distress with respect to the treatment of Parkinson's disease by means of the supplementary administration of nerve transmission substances and with respect to the treatment of dementia by means of the inhibitor against enzymes which decompose nerve transmission substances.

Against such background, a nerve nutrient factor including NGF has been in the limelight recently to the extent of even being taken up as a major research theme for a study on anti-dementia drugs.

In view of the facts that choline-working nerve cells in the fore-brain basal ganglion are much related to the memory function, that remarkable degeneration and deciduation of these cells are observed in Alzheimer's disease and that dementia may be likely to be induced for such causation, protection of choline-working nerve cells in the forebrain basal ganglion by means of the administration of nerve nutritive factors including NGF can be the therapy to directly eliminate the cause of dementia unlike the palliative anti-symptomatic therapy. Being in for the aged society, the development of drugs with such activities should be the urgent task of high priority.

A study on NGF is in the formost advance nowadays. The findings so far obtained by its administration into the cerebral ventricle revealed that NGF has an activity to protect the septal cells in a rat with the corpus fimbriatum incised, that it has an activity to bring recovery in labyrinth tests, and that it has an activity to improve the memory function in an aged rat to the extent of no abnormality.

However, the NGF molecular weight is so large that it cannot reach the inside of the brain by common means of the administration. The above activities can be found only when NGF has direct access to the inside of the brain, and such administration method must not be applied in clinical practice.

Under these circumstances, the advent of a drug with a nerve nutritive factor which can be useful for clinical treatment is expected.

The aim of the present invention is to demonstrate that new applications can be provided for a known product already in clinical use. The new applications are intended for prophylaxis or treatment of dementia on the basis of the new findings that this known product has activities to increase acetylcholine, a nerve transmission substance, and to grow neuroaxon. Furthermore, the clinical application of the known product is enabled by simple means of administration for protecting acetylcholine-working nerve cells in the brain which have a close connection with memory, and for maintaining the memory function. In addition, on the basis of the new findings that degeneration and deciduation of acetylcholine-working nerve cells in the brain of an animal with experimentally-induced dementia can be prevented, its application for prevention and treatment of dementia can be indicated.

It is well known that the nerve cells in Meinnert's basal nucleus, septal nucleus and hippocampus are closely related to the memory function through the communication network with the brain; it is especialy noticed that the deciduation of these choline-working nerve cells is remarkable in dementia cases of Alzheimer type. It is very important to maintain these nerve cells with the use of nerve nutritive factors for prevention of dementia, but such therapy has so far not been known. The present invention provides such application.

The effectiveness of the present invention was ascertained in comparison with NGF (neuroaxon growth factor) and other substances.

The drug of the present invention was administered intravenously through the intraperitoneal route into the septal nuclear cells with the supply of NGF (a neuroaxis growth factor: one of the nerve nutritive factors) discontinued from the hippocampus because of incision of fimbria fornix between the hippocampus and the septal necleus, and as a result, improvement was noted in degeneration and deciduation of septal nuclear cells.

The bioactive substance used in the present invention which is obtained from enzymatic hydrolysis of swine brain protein fraction is available from the applicants under the trade name Cerebrolysin. Each ml of this drug contains 3.00 mg of alanine, 0.25 mg of arginine, 3.00 mg of asparaginic acid, 0.06 g of cystine, 4.30 mg of glutaminic acid, 1.50 mg of glycine, 1.30 mg of histidine, 2.00 mg of isoleucine, 6.00 mg of leucine, 0.50 mg of methionine, 2.00 mg of phenylalanine, 2.00 mg of proline, 0.30 g of serine, 0.30 g of threonine, 0.50 g of tryptophane and 2.00 mg of thyrosine as amino acids as well as petides having a molecular weight of 10,000 or less; confer Erika Beyer (1980), Medizinische Welt, Vol. 31, No. 17, pages 636-637, and Chemical Abstracts Vol. 103, 1985, Page 94, Abstract 103:135149w.

Ganglion in the posterior root of the spinal nerve of chicken embryo was taken out of eggs aged 10-12 days, and suspended in a phosphoric acid buffer solution (PBS) containing no $Ca^{2+}$ and no $Mg^{2+}$ ions. It was treated with 0.1 % trypsin at 37° C for approximately 40 minutes, and centrifuged at 1,500 rpm for 5 minutes. Eagle's MEM medium was added to the cells thus collected with the supernatant removed, and the mixture was pipetted to disperse the cells. The suspensions of the cells thus obtained were filtered through a nylon mesh filter, transferred into a plastic resin dish, and incubated at 37° C for approximately 2 hours in a 5 % $CO_2$ incubator. Then, non-adhesive cells were collected in use for assay.

A bioactive substance Cerebrolysin was diluted in a phosphoric acid buffer solution (PBS) at a ratio of 10:100, 20:100, 40:100 and 80:100. For control purposes, the neuroaxis growth factor (NGF) was prepared at concentrations of 12.5 ng/100 µl, 25.0 ng/100 µl and 50.0 ng/100 µl, and also each amino acid contained in Cerebrolysin was suspended in a PBS (an amino acid solution) at the same concentrations as Cerebrolysin was. In addition, Solcoseryl (Taiho Pharmaceutical) was used, being diluted in a PBS at a ratio of 10:100, 20:100, 40:100 and 80:100.

Incidentally, this drug is administered at an amount of 1-30 ml directly by the intravenous route or through intravenous drip along with a physiological saline solution or a nutrition solution like a glucose-containing solution.

In another experiment, under anesthesia Wistar rats weighing 200-250 g were fixed on the brain fixing stand, and the fimbria fornix at either side was sucked for removal by the aspirator to cut off the route between the hippocampus and the septal nucleus.

The animals thus treated were divided into 5 groups from the aspect of the Cerebrolysin administration period; the 0-week group, the 1-week group, the 2-week group, the 3-week group and the 4-week group. After the administration thus designated was completed in each group, a cannula was inserted through the heart. Blood was removed with a physiological saline solution, and then, the brain was fixed, being refluxed with a 4% paraformaldehyde-phosphoric acid buffer physiological saline solution. The brain was, then, preserved in a cool place one overnight in a 10 % saccharose solution. A 40µm-thick thin slice was prepared and attached on a glass plate.

The slice thus obtained was treated with an anti-choline acetyl transferase, and colored by the ABC method. After a photographic picture was taken, the number of colored nerve cells was counted, and the size of such cells was measured.

This drug can be formulated in the manner of being injected as such intravenously at an amount of 1-30ml, or being dripped along with a physiological saline solution or a glucose solution, or being injected intramuscularly at an amount of 1-5 ml.

For more detailed explanation of the present invention, the following examples are given:

Example 1:

Ganglion in the posterior root of the spinal nerve was cut out from 2 chicken embryos aged 10-12 days, and gathered in 2 ml of PBS (phosphoric acid buffer solution which does not contain $Ca^{2+}$ and $Mg^{2+}$). Ganglion thus gathered was transferred into 5 ml of PBS containing 0.1 % trypsin, warmed at 37° C for 20 minutes, and then washed with Eagle's MEM medium containing 10 % semi-fetus serum (FCS). To the ganglion thus treated, 10 ml of Eagle's MEM medium was added, and the mixture was pipetted carefully 10-20 times to prepare cell suspensions. Then, 5 ml of the suspension was placed in each plastic resin dish having a dimension of 60 x 15 mm, and warmed at 37° C for 3 hours. The supernatant carefully collected was used as the ganglion cells in the posterior root of the spinal nerve of chicken embryo (DGC). DGC was prepared in 500 cells/0.9 ml, and placed in a 24-hole micro-plate treated with gelatine at an amount of 0.9 ml for each hole. Then, Cerebrolysin, NGF, Solcoseryl, mixed amino acids solution, dihydroergotin methylate, Cyticholine, calcium hopatenate, Dopamin and epinephrine, all of which concentrations had preliminarily been adjusted, were added individually to DGC. For blank purposes, 100 µl of PBS only was used. These samples were cultured at 37° C in a 5 % $CO_2$ incubator for 12 hours, 24 hours, 48 hours and 96 hours, and an observation was made by use of a phase microscope under 200-time enlargement. The following results were obtained:

In the case of the treatment with Cerebrolysin, a bioactive substance of the present invention in a range of concentrations of 10 µl/ml-80 µl/ml, neuroaxon was found to be produced from DGC. In a range of 10 µl/ml-20 µl/ml in particular, growth of neuroaxon was concentration-dependent. On the contrary, no sign of neuroaxon production was noted in the case of the treatment with Solcoseryl, mixed amino acids solution, dihydroergotin methylate, calcium hopatenate, Cyticholine, Dopamin and epinephrine. In NGF, neuroaxon production was observed, but it was inferior to the neuroaxon produced by the present invention in terms of growth mode (time and thickness). Thus, it is concluded that Cerebrolysin used in the present invention synthesizes a nerve nutrient factor inside nerve cells and that it contributes to neuroaxon production and growth.

| | Neuroaxon Production in DGC |
|---|---|
| **Present invention** | |
| Cerebrolysin | + |
| Controls | |
| Solcoseryl | − |
| Dihydroergotin methylate | − |
| Cyticholine | − |
| Epinephrine | − |

| | Neuroaxon Production in DGC |
|---|---|
| Calcium hopatentate | − |
| Dopamin | − |
| Mixed amino acids | − |
| NGF | + |

+: Produced            −: Not produced

Example 2:

Wistar rats weighing 200-250 g were fixed on the brain fixing stands under ketamine anesthesia at a rate of 5 mg/kg. The scalp was excised with a pair of scissors, and sterilized with hibitane. Then, the skull was shaved with a minister drill. Blood was blocked electrically, and the brain was exposed. Fimbria fornix was sucked for removal by the aspirator to cut off the route between the hippocampus and the sepal nucleus.

The animals thus treated were divided into 5 groups by the administration period; the 0-week group, the 1-week group, the 2-week group, the 3-week group and the 4-week group. Both Cerebrolysin and a physiological saline solution were injected intraperitoneally each at an amount of 5 mg/kg once a day to every group for the designated period.

After lapse of the designated administration period in each group, a cannula was inserted through the heart, and blood was removed with a physiological saline solution. Then, the brain was fixed, being refluxed with a 4 % paraformaldehydephosphoric acid buffer physiological saline solution. The cervical part was cut off to take out the brain, and it was, then, preserved in a cool place one overnight in a 10 % saccharose solution. A 40 µm-thick slice was prepared by use of the vibratome, and attached on a glass plate.

The slice thus obtained was treated with an anticholine acetyl transferase, and colored by the ABC method. After a photographic picture was taken, the number of colored nerve cells was counted, and the size of such cells was measured. The results obtained were as follows:

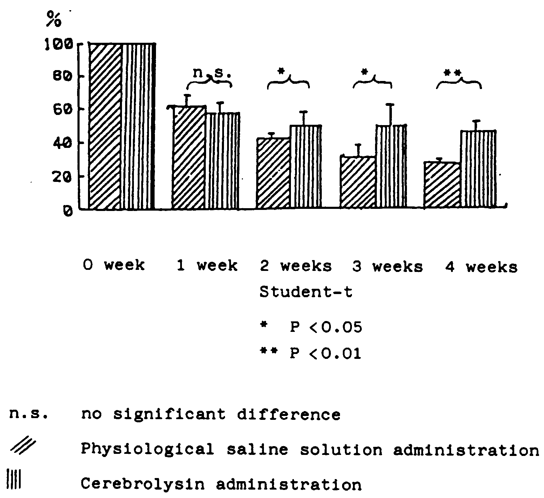

As shown in the attached Fig. 1, the activity to protect the septal cells turned out to be superior in the Cerebrolysin group to the control group in the 2nd week of the administration. At the time of the final evaluation, choline-working nerve cells were found to survive at a rate of 47 %; the survival rate was approximately 20 % higher in the Cerebrolysin group than in the control group.

In Fig. 1, incidentally, the survival of choline-working nerve cells is indicated along the Y-axis by percentage of those injured against those not injured.

In making an evaluation of the size of cells by a length as shown in Table 1, it is noted that the size of cells is markedly larger in the Cerebrolysin group than in the control group and that there is no particular difference in the size of cells between those injured and those not injured.

Table 1

| | Saline (5ml/kg) | Cerebrolysin (5ml/kg) | Student-t test |
|---|---|---|---|
| | $\bar{X} \pm SD$ | $\bar{X} \pm SD$ | |
| 1 week | $62 \pm 5.6$ | $57 \pm 4.4$ | No significant difference |
| 2 weeks | $42 \pm 0.4$ | $50 \pm 6.7$ | $P < 0.05$ |
| 3 weeks | $33 \pm 4.6$ | $50 \pm 10.9$ | $P < 0.05$ |
| 4 weeks | $27 \pm 0.9$ | $47 \pm 6.3$ | $P < 0.001$ |

Thus, Cerebrolysin exhibited an activity as a nerve nutritive factor through the intraperitoneal administration regarded clinically as being equal to the intravenous administration, and protected the septal cells, consequently improving the survival rate.

Claims

1. The use of an aqueous solution of the mixture of peptides with a molecular weight of 10,000 or less and amino acids, obtainable by enzymatic decomposition of swine brain protein fraction, in the preparation of a medicament for prophylaxis or treatment of dementia cases of Alzheimer type in mammals.

Patentansprüche

1. Verwendung einer wäßrigen Lösung des Gemisches aus Peptiden mit einem Molekulargewicht von 10.000 oder darunter und Aminosäuren, das durch enzymatischen Abbau der Schweinehirn-Proteinfraktion erhältlich ist, zur Herstellung eines Medikaments zur Prophylaxe oder Behandlung von Dementia-Fällen vom Alzheimer-Typ in Säugetieren.

Revendications

1. Utilisation d'une solution aqueuse d'un mélange de peptides ayant un poids moléculaire de 10000 ou moins et d'acides aminés, pouvant être obtenus par décomposition enzymatique d'une fraction protéique de cerveau de porc, pour la préparation d'un médicament pour la prophylaxie ou le traitement de cas de démence de type Alzheimer chez les mammifères.

0 week    1 week    2 weeks    3 weeks    4 weeks
Student-t

* P < 0.05
** P < 0.01

n.s.    no significant difference

/// Physiological saline solution administration

||| Cerebrolysin administration